**Europäisches Patentamt**

**European Patent Office**

**Office européen des brevets**

(19)

(11) Veröffentlichungsnummer: **0 278 377 B1**

(12)

# EUROPÄISCHE PATENTSCHRIFT

(45) Veröffentlichungstag der Patentschrift: **08.04.92**

(51) Int. Cl.⁵: **C07C 50/24**, C07C 46/06

(21) Anmeldenummer: **88101533.3**

(22) Anmeldetag: **03.02.88**

(54) **Verfahren zur Herstellung von Tetrachlor-1,4-benzochinon hoher Reinheit.**

(30) Priorität: **06.02.87 DE 3703567**

(43) Veröffentlichungstag der Anmeldung:
**17.08.88 Patentblatt  88/33**

(45) Bekanntmachung des Hinweises auf die
Patenterteilung:
**08.04.92 Patentblatt  92/15**

(84) Benannte Vertragsstaaten:
**BE CH DE ES FR GB IT LI NL SE**

(56) Entgegenhaltungen:
**DE-A- 2 645 114**

**BEILSTEINS HANDBUCH DER ORGANISCHEN
CHEMIE, 3. Ergänzungswerk, 4. Auflage,
Band 7, Teil 4, 1969, Springer Verlag Berlin**

(73) Patentinhaber: **HOECHST AKTIENGESELL-
SCHAFT
Postfach 80 03 20
W-6230 Frankfurt am Main 80(DE)**

(72) Erfinder: **Arndt, Otto, Dr.
Frankfurter Strasse 38
W-6238 Hofheim am Taunus(DE)**
Erfinder: **Papenfuhs, Theodor, Dr.
Heinrich-Bleicher-Strasse 40
W-6000 Frankfurt am Main 50(DE)**

## Beschreibung

Die vorliegende Erfindung betrifft ein Verfahren zur Herstellung von Tetrachlor-1,4-benzochinon (nachstehend als "Chloranil" bezeichnet) hoher Reinheit aus Hydrochinon.

Die Herstellung von Chloranil aus Hydrochinon (1,4-Dihydroxybenzol) oder 1,4-Benzochinon bzw. chloriertem 1,4-Benzochinon ist an sich nach folgenden in der Literatur beschriebenen Verfahren bekannt:

1. Chlorieren von Hydrochinon mit konzentrierter Salzsäure und konzentriertem Wasserstoffperoxid in Gegenwart von Magnesiumchlorid (DE-OS 2 645 114);

2. Einwirkung von Chlor auf Hydrochinon in siedender konzentrierter Salzsäure (Chemiker Zeitung 56 (1932), Seite 569);

3. Einwirkung von Salzsäure und Salpetersäure (Königswasser) auf Hydrochinon (J. Chem. Soc. Japan 63 (1942), Seite 1441);

4. Reaktionen von Antimon(V)-chlorid mit Hydrochinon (Chemiker Zeitung 104 (1980) Nr. 1, Seiten 13 und 14;

5. Einwirkung von Chlorwasserstoff, Luft und Metallsalzen auf Hydrochinon (DDR-Patentschrift Nr. 29 292);

6. Reaktion von Trichlor-1,4-Benzochinon mit Chlor bei Gegenwart von Jod und Wasser (Liebigs Annalen der Chemie, Supplementband 6 (1867), 213);

7. Behandlung eines Gemenges aus Trichlor-1,4-benzochinon und Tetrachlor-1,4-benzochinon mit Chlorwasserstoff in Eisessig und anschließende Einwirkung von konzentrierter Salpetersäure (Beilstein 7 637);

8. Einwirkung von konzentrierter Salzsäure und 35 % igem Wasserstoffperoxid auf 1,4-Benzochinon (Ann. Chimica applic. 22 (1932) 602;

9. Einleiten von Chlor in mit Chromtrioxid versetzte Lösung von Hydrochinon in wäßriger Salzsäure (Naugatuck Chem. Comp., USA, DRP 594 520, Friedländer 20, 2047, US-Patent 1 918 328).

Diese bekannten Verfahren haben jedoch folgende Nachteile:

Ad 1:    Es ist ein sehr großer Überschuß von Salzsäure (96fach molare Menge) erforderlich und es resultiert eine große Salzbelastung durch Zugabe von Magnesiumchlorid in der 7,4fach molaren Menge. Hinzu kommt, daß die angegebene zeitliche Temperaturführung bei der Zudosierung des Wasserstoffperoxids wegen der hohen Wärmetönung nicht eingehalten werden kann. Die Nacharbeitung dieses Verfahrens mit nur 30fach molarer Menge Salzsäure, 3fach molarer Menge Magnesiumchlorid und einer geeigneteren Temperaturführung ergab ein qualitativ schlechtes Chloranil (Fp. 215 - 220° C, Ausbeute 95 % der Theorie) mit Verunreinigungen an Trichlor-1,4-benzochinon und Tetrachlorhydrochinon.

Ad 2:    Das Eingasen von elementarem Chlor in siedende konzentrierte Salzsäure führt zu starkem Abgasen von elementarem Chlor in die Chlorwasserstoff-Brüden und macht die Anwendung eines großen Chlorüberschusses notwendig.

Die in der genannten Literaturstelle nicht angegebene Möglichkeit des Arbeitens unter Druck (im Autoklav) erfordert erhöhten technischen und sicherheitstechnischen Aufwand (voll emaillierte Armaturen, Ventile und Leitungsanschlüsse).

Die dort angegebene Menge von 37 %iger Salzsäure (28fach molare Menge) ist sehr hoch, reicht offenbar jedoch nicht aus, um ein bei 25° C rührbares Reaktionsgemisch zu erhalten. Das Gemisch erstarrt unter diesen Bedingungen unter Bildung von Tetrachlorhydrochinon.

Ad 3:    Bei diesem Verfahren wird eine etwa 25fach molare Menge Mineralsäure, zusammengesetzt aus 15fach molarer Menge Salzsäure und etwa 10fach molarer Menge Salpetersäure, eingesetzt. Die Ausbeute beträgt nur etwa 45 - 65 % der Theorie (Fp. 280° C). Über die Entsorgung des Säureüberschusses (besonders der Stickoxide) gibt es keine Hinweise.

Ad 4:    Die Verwendung von Antimon ist toxikologisch bedenklich und erfordert eine aufwendige Rückgewinnung. Außerdem soll bei diesem Verfahren Phosgen entstehen.

Ad 5:    In der genannten Patentschrift wird selbst auf den hohen Aufwand an Hilfsmitteln und die niedrige Ausbeute hingewiesen. Außerdem verbraucht die Wasserdampfdestillation viel Energie.

Ad 6:    Die Verwendung von Jod erschwert die Regeneration der Salzsäure zur Wiederverwertung.

Ad 7:    Der stufenweise Aufbau aus Chinon (hier: Trichlor-1,4-benzochinon) und Salzsäure mit anschließender Oxidation (hier: Salpetersäure) des chlorierten Hydrochinons (hier: Tetrachlorhydrochinon) zum Chinon mit den notwendigen zwischenstuflichen Reinigungsoperationen ist ein äußerst zeitraubendes Verfahren und ist für ein Betriebsverfahren ungeeignet.

Ad 8: Bei dem Verfahren wird zunächst mit konzentrierter Salzsäure (22 Bé = 37 %) (11fach molare Menge) 20 Stunden, anschließend mit Wasserstoffperoxid 35 % unterhalb von 60 ° C 12 Stunden behandelt. Man erhält zwar das Chloranil in einer hohen Ausbeute (Fp. 289 bis 290 ° C), durch die geringe Raum-Zeit-Ausbeute wird die Herstellung jedoch sehr teuer.

Ad 9: Bei dem Verfahren wird Hydrochinon in Salzsäure mit Chrom(VI)-oxid (20 g/Mol) zum Chinhydron oxidiert und dieses dann zunächst bei 25 ° C, schließlich in der Hitze mit elementarem Chlor zum Chloranil chloriert. Bei den heutigen Anforderungen des Umweltschutzes läßt sich die Anwendung von Chrom(VI)-oxid wirtschaftlich nicht mehr vertreten.

Der vorstehend gegebene Überblick über den Stand der Technik zeigt, daß die bekannten Verfahren im allgemeinen einen hohen Überschuß an Salzsäure, teilweise sogar umweltbelastende Hilfsstoffe und außergewöhnliche Oxidationsmittel sowie lange Reaktionszeiten benötigen.

Es wurde nun überraschenderweise gefunden, daß sich der Materialeinsatz (insbesondere an Salzsäure und Chlorierungsmittel) und der Zeitaufwand erheblich verringern lassen, und sich ferner ein Produkt hoher Reinheit erzielen läßt, wenn man Chlorierung und Oxidation zeitlich und temperaturmäßig so gewichtet, daß bis zur Einführung von 2 Chloratomen eine Oxidation zu Chinhydron oder Chinon vermieden wird, wodurch die Einführung des dritten Chloratoms zeitgleich mit der Aufoxidation zum Trichlorbenzochinon erfolgt.

Gegenstand der Erfindung ist somit ein verbessertes Verfahren zur Herstellung von Tetrachlor-1,4-benzochinon hoher Reinheit, in dem man auf 1 Mol Hydrochinon in der mindestens 12fach, vorzugsweise 12fach molaren Menge 30 bis 37 %iger Salzsäure, vorzugsweise 37 %iger Salzsäure, die 3,8- bis 4,2fach, vorzugsweise 4fach molare Menge 30 bis 37 %iger Salzsäure, vorzugsweise 37 %iger Salzsäure, und die 1,9- bis 2,1fach, vorzugsweise 2fach molare Menge 50 bis 35 %igen Wasserstoffperoxids, vorzugsweise 35 %igen Wasserstoffperoxids, bei 5 bis 50 ° C, vorzugsweise 10 bis 20 ° C, einwirken läßt, dann auf die angefallene, auf 45 bis 55 ° C eingestellte, im wesentlichen das 2,5-Dichlorhydrochinon enthaltende Suspension wiederum die 3,8- bis 4,2fach, vorzugsweise 4fach molare Menge 30 bis 37 %iger Salzsäure, vorzugsweise 37 %iger Salzsäure, und die 1,9- bis 2,1fach, vorzugsweise 2fach molare Menge 50 bis 35 %igen Wasserstoffperoxids, vorzugsweise 35 %igen Wasserstoffperoxids, jeweils bezogen auf das eingesetzte Hydrochinon, bei 50 bis 95 ° C einwirken läßt, und schließlich zu der im wesentlichen aus dem Trichlor-1,4-benzochinon bestehenden Suspension die 1,9- bis 2,1fach, vorzugsweise 2fach molare Menge Salzsäure des genannten Konzentrationsbereichs, vorzugsweise 37 %iger Konzentration, und die 0,95- bis 1,05fach, vorzugsweise 1fach molare Menge Wasserstoffperoxid des genannten Konzentrationsbereichs, vorzugsweise 35 %iger Konzentration, jeweils bezogen auf das eingesetzte Hydrochinon, bei 95 bis 115 ° C, vorzugsweise 105 ° C, so langsam zugibt, daß kein Chlor abgast.

Der Grund für die verbesserte Verfahrensführung ist die Ausnutzung der höheren Löslichkeiten der 1- bis 3fach chlorierten Hydrochinone in der konzentrierten Salzsäure im Vergleich zu den entsprechend 1- bis 3fach chlorierten 1,4-Benzochinonen.

Abweichungen vom erfindungsgemäßen Verfahren dokumentieren sich in erhöhtem Verbrauch von Chlorierungsmittel und schlechter Qualität des Chloranils (erhöhter Gehalt an Tetrachlorhydrochinon, 2,3- und 2,5-Dichlor-1,4-benzochinon, Trichlor-1,4-Benzochinon und unbekannten Nebenkomponenten) (erfaßt durch HPLC = high performance liquid chromatography und HPTLC = high performance thin layer chromatography).

Der durch die höheren Löslichkeiten der 1- bis 3fach chlorierten Hydrochinone bedingte raschere Umsatz mit dem Chlorierungsmittel wird durch Verwendung von säure- und chlorstabilen oberflächenaktiven Hilfsmitteln noch unterstützt, wobei diese zudem ein mögliches Schäumen der Reaktionsmischung wirkungsvoll unterbinden. Als derartiges Hilfsmittel eignet sich bevorzugt sekundäres Alkansulfonat. Die oberflächenaktiven Hilfsmittel werden zweckmäßigerweise in einer Menge von etwa 5 bis 15 Millimol pro Mol eingesetztem Hydrochinon angewendet.

Von großer Bedeutung ist auch die Konzentration der Salzsäure, insbesondere zu Beginn der Chlorierung. Bei Startkonzentrationen von Salzsäure unterhalb von 30 % werden stark gefärbte Reaktionsmischungen erhalten, die schließlich ein Chloranil schlechter Qualität ergeben. Durch eine ausreichend hohe Startkonzentration der Salzsäure wird das Reduktionspotential der Hydrochinone so weit herabgesetzt, daß die im Sinn der Erfindung bis zu Einführung des dritten Chloratoms von einer zu frühzeitigen Oxidation zum 1,4-Benzochinon bewahrt bleiben.

Die Salzsäure-Konzentration nimmt während des Umsatzes ab. Hierbei endet die Reaktion mit einer 20 %igen, als Azeotrop regenerierbaren Salzsäure, wenn man mit einer 37 %igen Salzsäure startet.

Das erfindungsgemäße Verfahren ist im Vergleich zum Stand der Technik ökonomisch und ökologisch vorteilhaft. Die Mutterlaugen werden auf 20 %ige Salzsäure destillativ regeneriert. Die Regeneratsäuren sind farblos, enthalten höchstens Spuren organischen Kohlenstoffs und können an anderer Stelle der Produktion wieder eingesetzt werden. Außer dem Waschfiltrat und einem im wesentlichen aus sekundärem Alkansulfat bestehenden Destillationsrückstand fallen keine weiteren zu entsorgenden Fabrikationsrückstände an.

Das erfindungsgemäß hergestellte Chloranil ist von hoher Reinheit, was sich am Schmelzpunkt (281 -282° C) und daran, daß es kein Tetrachlorhydrochinon enthält, zeigt.
C-Gehalt: 29,3 - 29,7 % (theoretisch 29,31 %)
Cl-Gehalt: 57,3 - 57,6 % (theoretisch 57,67 %)
Reingehalt (titanometrisch) = 100,0 %
Nachstehend sei noch eine bevorzugte Ausführung des erfindungsgemäßen Verfahrens angegeben, wobei Teile Gewichtsteile sind:
1 Teil Hydrochinon wird in 10,6 Teilen Salzsäure 37 % (entspricht der 12fach molaren Menge, bezogen auf das eingesetzte Hydrochinon) in Gegenwart von ca. 0,025 Teilen sekundärem Alkansulfonat bei 10° C zunächst mit nur ca. 2 Teilen Wasserstoffperoxid 35 % (entspricht der 2fach molaren Menge, bezogen auf das eingesetzte Hydrochinon) umgesetzt. Die Reaktion ist stark exotherm. Es bildet sich eine weiße Suspension aus
64 Mol-% 2,5-Dichlorhydrochinon,
23 Mol-% 2,3-Dichlorhydrochinon und
13 Mol-% 2-Chlorhydrochinon.

Die Suspension wird auf 50° C geheizt. Unter weiterem Aufheizen bis auf 95° C werden nochmals ca. 2 Teile Wasserstoffperoxid 35 % zudosiert, wobei die Reaktion zunächst immer noch exotherm ist. Die Suspension verändert ihre Färbung von Weiß nach Hellbraun. Das an dieser Stelle auftretende Schäumen wird durch die Anwesenheit des sekundären Alkansulfonats wirkungsvoll unterdrückt. In dieser Phase entstehen nahezu gleichzeitig nebeneinander das 2,3,5-Trichlorhydrochinon und 2,3,5-Trichlor-1,4-benzochinon. Die Einführung des 4 Chloratoms erfolgt dann bei 105° C mit ca. 1 Teil Wasserstoffperoxid 35 %. Insgesamt werden ca. 5,3 Mol Wasserstoffperoxid, bezogen auf das eingesetzte Hydrochinon, eingesetzt.

Nach Filtrieren und Waschen erhält man reines Chloranil in einer Ausbeute von 98 % der Theorie, bezogen auf eingesetztes Hydrochinon. Das Abgas enthält nur Spuren von elementarem Chlor, die dem zuletzt zudosierten überschüssigen Wasserstoffperoxid entsprechend (ca. 3 bis 4 Mol-%, bezogen auf eingesetztes Hydrochinon). Es enthält keinen Chlorwasserstoff. Die ca. 17 bis 20 %ige Mutterlauge wird auf eine ca. 18 bis 20 %ige Destillatsalzsäure aufgearbeitet. Als Fabrikationsrückstand fällt nur das eingesetzte sekundäre Alkansulfonat (Destillationsrückstand bei Salzsäure-Regeneration) und das Waschfiltrat an. Letzteres kann biologisch gereinigt werden (Rest-CSB (Chemischer Sauerstoffverbrauch) = 6,1 kg Sauerstoff ($O_2$) pro Tonne Chloranil).

Chloranil ist ein wertvolles Zwischenprodukt zur Herstellung von Farbstoffen und Schädlingsbekämpfungsmitteln. Ferner wird es als Photochemikalie und Vulkanisationsmittel eingesetzt und dient als Zusatz für Schmiermittel.

Das nachstehende Beispiel und das angegebene Vergleichsbeispiel dienen zur näheren Erläuterung der Erfindung.

**Beispiel**

Zu einem Gemisch aus 592 Teilen Salzsäure 37 % (6,0 Mol), 55,6 Teilen Hydrochinon (0,5 Mol) und 1,4 Teilen sekundärem n-Alkansulfonat (z.B. $C_{13-17}$) werden bei 10° C unter Außenkühlung (5° C) 100 Teile Wasserstoffperoxid 35 % (1,0 Mol) in 120 Minuten zugegeben. Anschließend wird 30 Minuten bei 10° C nachgerührt. Dann wird in 30 Minuten gleichmäßig auf 50° C aufgeheizt. Danach werden zu der weißen Suspension (Dichlorhydrochinon) in 60 Minuten erneut 100 Teile Wasserstoffperoxid 35 % zudosiert, wobei man gleichzeitig zunächst durch die Wärmetönung, schließlich durch äußeres Heizen die Temperatur auf 95° C steigen läßt. Die nun hellbraune, dünne, nicht schäumende Suspension wird dann in 60 Minuten auf 105° C aufgeheizt. Gleichzeitig werden 25 Teile Wasserstoffperoxid 35 % (0,25 Mol) zugegeben (Heizbad 110° C). Die Suspension ist jetzt hellgelb. Man rührt 240 Minuten bei 105° C nach, wobei je nach Umsatzfortschritt (das Fortschreiten der Chlorierung und Oxidation wird mit HPTLC verfolgt) noch bis maximal 35 Teile Wasserstoffperoxid 35 % (0,36 Mol) zugegeben werden müssen. Die Chlorierzeit beträgt 9 Stunden.

Nach Beendigung des Umsatzes spült man das restliche in der Reaktoratmosphäre vorhandene Chlorgas in eine Vorlage aus 150 Teilen Wasser und 150 Teilen Natronlauge 33 % (0,02 Mol $Cl_2$). Das Abgas enthält keine Salzsäure.

Nach Filtration bei 90 bis 95° C und Waschen mit 600 Teilen Wasser erhält man 121 Teile reines Chloranil (0,49 Mol).
C-Gehalt: 29,3 - 29,7 % (theoretisch 29,31 %)
Cl-Gehalt: 57,3 - 57,6 % (theoretisch 57,67 %)
Reingehalt (titanometrisch) = 100 %
Schmelzpunkt: 281 - 282° C.

Die Mutterlauge (781 Teile 17 %ige Salzsäure) wird bei Normaldruck auf Rückstand destilliert. Man erhält 724 Teile Salzsäure 18 % (farblos, C-organisch = 75 mg/l) und 6,0 Teile Destillationsrückstand, der sich mit Wasser aus dem Destillationskolben entfernen läßt. Das Waschfiltrat läßt sich biologisch reinigen.

**Vergleichsbeispiel**

Man verfährt, wie im Beispiel beschrieben, jedoch mit dem Unterschied, daß bei 10°C statt nur 100 Teile 170 Teile Wasserstoffperoxid 35 % (1,75 Mol) in 240 Minuten zugesetzt werden. Man erhält eine dunkelviolette Suspension. Nach dem Erwärmen auf 20°C heizt man in 60 Minuten auf 50°C und gibt gleichzeitig 24 Teile Wasserstoffperoxid 35 % (0,25 Mol) hinzu. Die Suspension verfärbt sich über Graubraun, Olivfarben nach Gelb.

Nach weiterer Zugabe von 36 Teilen Wasserstoffperoxid 35 % bei 50 bis 95°C (45 Minuten), 36 Teilen bei 95°C (60 Minuten), 36 Teilen bei 95 bis 105°C (360 Minuten) (insgesamt 302 Teile) und der üblichen Aufarbeitung erhält man 120 Teile Chloranil vom Schmelzpunkt 281 bis 282°C mit einem zu niedrigen Chlorgehalt von 56,4 % (theoretisch 57,7 %).

**Patentansprüche**

1. Verfahren zur Herstellung von Tetrachlor-1,4-benzochinon hoher Reinheit durch Einwirkung von Wasserstoffperoxid und Salzsäure auf Hydrochinon, dadurch gekennzeichnet, daß man auf 1 Mol Hydrochinon in der mindestens 12fach molaren Menge 30 bis 37 %iger Salzsäure die 3,8- bis 4,2fach molare Menge 30 bis 37 %iger Salzsäure und die 1,9- bis 2,1fach molare Menge 50 bis 35 %igen Wasserstoffperoxids bei 5 bis 50°C einwirken läßt, dann auf die angefallene, auf 45 bis 55°C eingestellte, im wesentlichen das 2,5-Dichlorhydrochinon enthaltende Suspension wiederum die 3,8- bis 4,2fach molare Menge 30 bis 37 %iger Salzsäure und die 1,9- bis 2,1fach molare Menge 50 bis 35 %igen Wasserstoffperoxids, jeweils bezogen auf das eingesetzte Hydrochinon, bei 50 bis 95°C einwirken läßt, und schließlich zu der nunmehr im wesentlichen aus dem Trichlor-1,4-benzochinon bestehenden Suspension die 1,9- bis 2,1fach molare Menge Salzsäure des genannten Konzentrationsbereichs und die 0,95- bis 1,05fach molare Menge Wasserstoffperoxid des genannten Konzentrationsbereichs, jeweils bezogen auf das eingesetzte Hydrochinon, bei 95 bis 115°C, so langsam zugibt, daß kein Chlor abgast.

2. Verfahren nach Anspruch 1, dadurch gekennzeichnet, daß man in Gegenwart eines säure- und chlorstabilen oberflächenaktiven Mittels arbeitet.

3. Verfahren nach Anspruch 1 oder 2, dadurch gekennzeichnet, daß man in Gegenwart eines sekundären Alkansulfonats arbeitet.

**Claims**

1. A process for the preparation of high-purity tetrachloro-1,4-benzoquinone by the action of hydrogen peroxide and hydrochloric acid on hydroquinone, which comprises causing 3.8 to 4.2 times the molar quantity of 30 to 37% hydrochloric acid and 1.9 to 2.1 times the molar quantity of 50 to 35% hydrogen peroxide to act at 5 to 50°C on 1 mole of hydroquinone in at least 12 times the molar quantity of 30 to 37% hydrochloric acid, then adjusting the resulting suspension, which essentially contains 2,5-dichlorohydroquinone, to 45 to 55°C and again causing to act on it, at 50 to 95°C, 3.8 to 4.2 times the molar quantity of 30 to 37% hydrochloric acid and 1.9 to 2.1 times the molar quantity of 50 to 35% hydrogen peroxide, in each case based on the hydroquinone used, and finally adding to the suspension, which is now essentially composed of trichlorol-1,4-benzoquinone, 1.9 to 2.1 times the molar quantity of hydrochloric acid of the said concentration range, and 0.95 to 1.05 times the molar quantity of hydrogen peroxide of the said concentration range, in each case based on the hydroquinone used, at 95 to 115°C, sufficiently slowly for no chlorine to escape.

2. The process as claimed in claim 1, which is carried out in the presence of a surface-active agent which is stable to acid and chlorine.

3. The process as claimed in claim 1 or 2, which is carried out in the presence of a secondary alkanesulfonate.

**Revendications**

1. Procédé pour préparer la tétrachlorobenzoquinone-1,4 à l'état très pur par action de peroxyde d'hydrogène et d'acide chlorhydrique sur l'hydroquinone, procédé caractérisé en ce qu'on fait agir sur 1 mole d'hydroquinone, dans une quantité d'acide chlorhydrique à 30-37 % représentant au moins 12 fois la quantité molaire, une quantité d'acide chlorhydrique à 30-37 % représentant de 3,8 à 4,2 fois la quantité molaire, et une quantité de peroxyde d'hydrogène à 50-35 % représentant de 1,9 à 2,1 fois la quantité molaire, à une température de 5 à 50 °C, puis on fait à nouveau agir, sur la suspension obtenue, portée à une température de 45 à 55°C, qui contient essentiellement la dichloro-2,5 hydroquinone, une quantité d'acide chlorhydrique à 30-37 % représentant de 3,8 à 4,2 fois la quantité molaire et une quantité de peroxyde d'hydrogène à 50-35 % représentant de 1,9 à 2,1 fois la quantité molaire, à chaque fois par rapport à l'hydroquinone mise en jeu, à une température de 50 à 95°C, et enfin on ajoute à la suspension, qui est essentiellement constituée à ce moment de trichloro-benzoquinone-1,4, une quantité d'acide chlorhydrique, dont la concentration est située dans l'intervalle indiqué, représentant de 1,9 à 2,1 fois la quantité molaire et une quantité de peroxyde d'hydrogène, dont la concentration est située dans l'intervalle indiqué, représentant de 0,95 à 1,05 fois la quantité molaire, à chaque fois par rapport à l'hydroquinone mise en jeu, à une température de 95 à 115°C, cette addition étant effectuée suffisamment lentement pour qu'il ne séchappe pas de chlore.

2. Procédé selon la revendication 1 caractérisé en ce qu'on opère en présence d'un surfactif stable en présence d'acides et de chlore.

3. Procédé selon l'une des revendications 1 et 2, caractérisé en ce qu'on opère en présence d'un alcanesulfonate secondaire.